# EUROPÄISCHE PATENTSCHRIFT

(11) **EP 2 268 460 B1**
(45) Veröffentlichungstag und Bekanntmachung des Hinweises auf die Patenterteilung: **21.10.2020**
(21) Anmeldenummer: 09733169.8
(22) Anmeldetag: 17.04.2009
(51) Int. Cl.: B25J 9/16, A61B 6/00, A61B 6/10

(54) **RÖNTGENVORRICHTUNG UND MEDIZINISCHER ARBEITSPLATZ**
X-RAY DEVICE AND MEDICAL WORKPLACE
DISPOSITIF DE RADIOGRAPHIE ET POSTE DE TRAVAIL MÉDICAL

(30) Priorität: 17.04.2008 DE 102008019345
(43) Veröffentlichungstag der Anmeldung: 05.01.2011
(73) Patentinhaber: KUKA Deutschland GmbH, 86165 Augsburg (DE)
(72) Erfinder: ORTMAIER, Tobias, Prof. Dr.-In.g, 30966 Hemmingen (DE); HEILIGENSETZER, Peter, 86152 Augsburg (DE)
(74) Vertreter: Böss, Dieter Alexander
(86) Internationale Anmeldenummer: PCT/EP2009/054576
(87) Internationale Veröffentlichungsnummer: WO 2009/127713

(56) Entgegenhaltungen:
- EP-A2- 0 087 198
- DE-A1- 10 200 534
- US-A- 5 485 502
- TIM SCHRÖDER: "High-sensitivity Robot Arms" SIEMENS MEDICAL MAGAZINE, [Online] Oktober 2006 (2006-10), Seiten 62-64, XP002547865 Gefunden im Internet: URL:http://www.medical.siemens.com/siemens /en_GB/rg_marcom_FBAs/files/brochures/News /Positioning_Robots_Medical_Solutions_Oct_ 2006_62-64_e.pdf> [gefunden am 2009-09-29]

## Beschreibung

Die Erfindung betrifft eine Röntgenvorrichtung und einen medizinischen Arbeitsplatz.

Roboter im Allgemeinen sind Arbeitsmaschinen, die zur automatischen Handhabung und/ oder Bearbeitung von Objekten mit Werkzeugen ausgerüstet werden können und in mehreren Bewegungsachsen beispielsweise hinsichtlich Orientierung, Position und Arbeitsablauf programmierbar sind. Roboter weisen üblicherweise programmierbare Steuerungen (Steuerungsvorrichtungen) auf, die während des Betriebs die Bewegungsabläufe des Roboters steuern.

Roboter werden zunehmend in der Medizintechnik z.B. als Träger von Patientenlagerungs- und Diagnosesystemen, wie beispielsweise Röntgenvorrichtungen, eingesetzt. Hierbei bewegt sich der Roboter in direktem Kontakt oder unmittelbaren Kontakt zu mit Personen, wie beispielsweise einem Arzt oder einem Patienten, ohne trennende Schutzeinrichtungen, wie es insbesondere bei industriellen Anwendungen üblich ist. Damit der Roboter nicht versehentlich eine Person verletzt, kommt der Sicherheitstechnik eine entscheidende Bedeutung zu. So kann z.B. die Bewegung des Roboters in sicherer Technik überwacht werden. Die Überwachung umfasst u.A. einfehlersichere Winkelgeber, redundante Berechnung von Position, Geschwindigkeit und Beschleunigung, sowie eine sichere Überwachung der abgeleiteten Größen.

Die DE 10 2005 012 700 A1 offenbart eine Röntgenvorrichtung, aufweisend einen Roboter mit sechs Drehachsen und einen am Roboter befestigten U-förmigen Trägern, an dem eine Röntgenstrahlenquelle und ein Röntgendetektor angeordnet sind.

Die DE 10 2005 041 606 A1 offenbart eine Patientenpositioniervorrichtung zum Positionieren eines Patienten in einer Bestrahlungsposition für eine Strahlentherapieanlage. Die Patientenpositioniervorrichtung umfasst ein Patientenhalterungsmodul und einen das Patientenhalterungsmodul bewegenden Positionierarm, dessen Bewegung ein Therapiekontrollzentrum steuert. Am Patientenhalterungsmodul sind Drucksensoren angeordnet, so dass ein mittels des Patientenhalterungsmoduls bewegter Patient gegen ungewollte Einwirkungen über eine Unterbrechung der Ansteuerung des Positionierarms bei Aktivierung eines der Drucksensoren gesichert ist.

Die DE 102 00 534 A1 und die EP 0 087 198 A2 offenbaren jeweils eine medizinische Untersuchungs- und/oder Behandlungseinrichtung. Diese umfasst ein bodenseitiges Stativ an dem ein Träger um eine Achse drehbar gelagert ist. Der Träger weist zwei weitere Teilträger auf, an denen jeweils eine Strahlungsquelle oder ein Strahlungsempfänger angeordnet ist. Die medizinische Untersuchungs- und/oder Behandlungseinrichtung umfasst außerdem einen Patientenlagerungstisch mit einer höhenverstellbaren und in drei Orientierungen schwenkbaren Tischplatte.

Eine Aufgabe der Erfindung ist es, eine Röntgenvorrichtung mit einem Roboter und mit einer am Roboter angeordneten, eine Röntgenstrahlenquelle und einen Röntgenstrahlenempfänger umfassenden Tragevorrichtung für einen medizinischen Arbeitsplatz anzugeben, so dass zumindest die Gefahr einer Kollision zwischen der Röntgenvorrichtung und einem sich am medizinischen Arbeitsplatz aufhaltenden Lebewesen verringert wird.

Eine weitere Aufgabe der Erfindung ist es, einen medizinischen Arbeitsplatz mit einer Röntgenvorrichtung, die einen Roboter und eine am Roboter angeordnete, eine Röntgenstrahlenquelle und einen Röntgenstrahlenempfänger umfassende Tragevorrichtung aufweist, anzugeben, so dass zumindest die Gefahr einer Kollision zwischen der Röntgenvorrichtung und einem sich am medizinischen Arbeitsplatz aufhaltenden Lebewesen verringert wird.

Die Aufgabe der Erfindung wird gelöst durch eine Röntgenvorrichtung für einen medizinischen Arbeitsplatz, aufweisend:
- einen Roboter, aufweisend mehrere Achsen, eine Steuerungsvorrichtung, die eingerichtet ist, die Achsen für eine Bewegung des Roboters anzusteuern, und eine Befestigungsvorrichtung, und
- eine an der Befestigungsvorrichtung angeordnete, eine Röntgenstrahlenquelle und einen Röntgenstrahlenempfänger aufweisende Tragevorrichtung,
wobei in der Steuerungsvorrichtung ein 3D-Modell von dem Roboter mit angeordneter Tragevorrichtung gespeichert ist, das die räumliche Ausdehnung des Roboters mit angeordneter Tragevorrichtung während der Bewegung des Roboters modelliert,
wobei das 3D-Modell auch die räumliche Ausdehnung eines sich innerhalb des medizinischen Arbeitsplatzes befindlichen Lebewesens modelliert und die Steuerungsvorrichtung aufgrund des 3D-Modells eine potenzielle Kollision der Röntgenvorrichtung mit dem Lebewesen erkennt und den Roboter veranlasst, eine Maßnahme zum Vermeiden der potenziellen Kollision einzuleiten, des Weiteren aufweisend wenigstens einen mit der Steuerungsvorrichtung gekoppelten Sensor, mittels dem die Umgebung der Röntgenvorrichtung erfasst wird und der eingerichtet ist, eine Bewegung des Lebewesens zu erkennen, um die aktuelle Position des Lebewesens im 3D-Modell zu aktualisieren.

Die erfindungsgemäße Röntgenvorrichtung weist demnach den Roboter mit mehreren Achsen auf, die von der Steuerungsvorrichtung im Betrieb angesteuert werden. Dies wird z.B. realisiert, indem der Roboter Antriebe aufweist, die die Steuerungsvorrichtung ansteuern, so dass die Antriebe die Achsen bewegen. Die Antriebe sind beispielsweise elektrische Antriebe.

Der Roboter weist ferner die Befestigungsvorrichtung, z.B. einen Flansch, auf, an der die Tragevorrichtung angeordnet ist. Die Tragevorrichtung, die z.B. C- oder U-förmig ausgebildet ist, weist wiederum die Röntgenstrahlenquelle und den Röntgenstrahlenempfänger auf. Die Röntgenstrahlenquelle und der Röntgenstrahlenempfänger sind derart an der Tragevorrichtung angeordnet, dass im Betrieb der erfindungsgemäßen Röntgenvorrichtung eine von der Röntgenstrahlungsquelle erzeugte Röntgenstrahlung auf ein Lebewesen trifft, von diesem teilweise geschwächt wird und auf dem Röntgenstrahlenempfänger auftrifft. Der Röntgenstrahlenempfänger, der z.B. ein Röntgenbildverstärker oder ein Flachbilddetektor ist, wandelt z.B. die auftreffende Röntgenstrahlung in elektrische Signale um, deren Verteilung als Röntgenbild darstellbar ist.

Im Betrieb der erfindungsgemäßen Röntgenvorrichtung bewegt der Roboter die Tragevorrichtung, um z.B. eine oder mehrere Röntgenbilder von einem Lebewesen aufzunehmen. Das Lebewesen kann z.B. auf der Patientenliege liegen oder sitzen und der Roboter bewegt automatisch die Tragevorrichtung zum Lebewesen, um das oder die Röntgenbilder herzustellen. Die erfindungsgemäße Röntgenvorrichtung kann insbesondere derart ausgeführt sein, dass sie eine Serie von 2D-Projektionen vom Lebewesen aufnimmt, aus denen für den Fachmann in bekannter Weise ein Volumendatensatz vom Lebewesen errechnet werden kann. Ist die Tragevorrichtung als C-Bogen ausgeführt, kann der Roboter den C-Bogen während der Aufnahme der 2D-Projektionen insbesondere längs seines Umfangs (Orbitalbewegung) oder während einer Angulationsbewegung verstellen. Andere Bewegungen sind aufgrund des Roboters ebenfalls möglich.

Um bei der Bewegung des Roboters eine Kollision mit dem Lebewesen zu vermeiden, ist in der Steuerungsvorrichtung des Roboters das 3D-Modell hinterlegt. Das 3D-Modell modelliert die räumlichen Abmessungen des Roboters und der die Röntgenstrahlenquelle und den Röntgenstrahlenempfänger aufweisenden Tragevorrichtung. Beispielsweise aufgrund der Achsenstellungen des Roboters während seiner Bewegung ist es der Steuerungsvorrichtung möglich, das (dreidimensionale) 3D-Modell vom Roboter mit Tragevorrichtung der aktuellen Ausrichtung im Raum zu aktualisieren.

Das 3D-Modell modelliert zusätzlich die räumliche Ausdehnung eines Lebewesens, das beispielsweise das Lebewesen ist, von dem das oder die Röntgenbilder aufgenommen werden, oder ein weiters Lebewesen, wie z.B. ein behandelnder Arzt. Somit ist es der Steuerungsvorrichtung möglich, aufgrund des 3D-Modells eine potenzielle Kollision der Röntgenvorrichtung mit dem Lebewesen vorzeitig zu erkennen und die Maßnahme zum Vermeiden der potenziellen Kollision einzuleiten. Diese Maßnahme umfasst z.B. eine Notbremsung der Bewegung des Roboters, eine

Verlangsamung seiner Bewegung oder auch eine Änderung seiner Trajektorie, um dem Lebewesen auszuweichen.

Ein weiterer Aspekt der Erfindung betrifft einen medizinischen Arbeitsplatz, aufweisend
- eine erfindungsgemäße Röntgenvorrichtung, wie oben schon definiert,
- einen zweiten Roboter, der mehrere zweite Achsen, eine zweite Steuerungsvorrichtung, die eingerichtet ist, die zweiten Achsen für eine Bewegung des zweiten Roboters anzusteuern, und eine zweite Befestigungsvorrichtung aufweist,
- eine an der zweiten Befestigungsvorrichtung angeordnete Patientenliege und
- eine Rechenvorrichtung, in der ein 3D-Modell vom medizinischen Arbeitsplatz gespeichert ist, das sowohl das Modell des ersten Roboters mit angeordneter Tragvorrichtung und des sich innerhalb des medizinischen Arbeitsplatzes befindlichen Lebewesens enthält, als auch die räumliche Ausdehnung des zweiten Roboters mit angeordneter Patientenliege während der Bewegung des weiteren Roboters modelliert, wobei die Rechenvorrichtung aufgrund des 3D-Modells eine potenzielle Kollision der Röntgenvorrichtung mit dem weiteren Roboter und/oder mit der Patientenliege erkennt und die beiden Roboter veranlasst, eine Maßnahme zum Vermeiden der potenziellen Kollision einzuleiten.

Der erfindungsgemäße weitere Arbeitsplatz weist demnach im Wesentlichen die erfindungsgemäße Röntgenvorrichtung und zusätzlich den weiteren Roboter auf, an dessen Befestigungsvorrichtung, die z.B. ein Flansch ist, die Patientenliege befestigt ist. Mittels des weiteren Roboters kann demnach die Position und/oder die Orientierung der Patientenliege im Raum geändert werden.

Der weitere medizinische Arbeitsplatz weist ferner die Rechenvorrichtung auf, in der das 3D-Modell vom Arbeitsplatz gespeichert ist. Die erste Steuervorrichtung kann die Rechenvorrichtung umfassen, wobei die beiden Steuerungsvorrichtungen miteinander gekoppelt sind. Es ist aber auch möglich, dass in beiden Steuerungsvorrichtungen das 3D-Modell vom medizinischen Arbeitsplatz gespeichert ist. Aufgrund der Kopplung der Steuerungsvorrichtungen kann dann das bzw. die 3D-Modelle der Bewegung der Roboter angepasst werden.

Die Rechenvorrichtung ist eingerichtet, aufgrund des 3D-Modells eine potenzielle Kollision der Röntgenvorrichtung, des weiteren Roboters und/oder der Patientenliege mit dem Lebewesen zu erkennen und die beiden Roboter zu veranlassen, eine Maßnahme zum Vermeiden der potenziellen Kollision einzuleiten.

Die erfindungsgemäße Röntgenvorrichtung weist wenigstens einen mit der Steuerungsvorrichtung gekoppelten Sensor auf, der eingerichtet ist, eine Bewegung des Lebewesens zu erkennen, um die aktuelle Position des Lebewesens im 3D-Modell zu aktualisieren. Mittels des wenigstens einen Sensors kann die Umgebung der erfindungsgemäßen Röntgenvorrichtung erfasst werden. Dies ist dann beispielsweise vorteilhaft, wenn eine Bewegung oder eine Veränderung der Position der weiteren Vorrichtung des medizinischen Arbeitsplatzes nicht planbar oder vorhersagbar ist. Mittels der von dem Sensor stammenden Signale ist es dann möglich, das 3D-Modell entsprechend zu aktualisieren. Die Verwendung des wenigstens einen Sensors ist auch zur Überwachung des Lebewesens geeignet, insbesondere dann, wenn dieses sich bewegt. Handelt es sich z.B. bei dem Lebewesen um einen auf der Patientenliege insbesondere liegenden Patienten, von dem die erfindungsgemäße Röntgenvorrichtung ein Röntgenbild herstellen soll, dann kann somit zumindest die Gefahr einer Kollision mit dem Lebewesen, wenn dieses sich z.B. versehentlich bewegt, verringert werden. Der Sensor kann z.B. ein optischer Sensor sein, mittels dem die Position und/oder Bewegung des Lebewesens erfasst wird.

Als Sensor kann beispielsweise ein Kontaktsensor, ein Nahfeldsensor und/oder ein Weitfeldsensor verwendet werden.

Werden beispielsweise mehrere Sensoren verwendet, dann kann ein mittels der Sensoren überwachter Messbereich folgendermaßen untergliedert werden:
Kontaktsensoren, die beispielsweise als relativ einfache Schalter ausgeführt sein können, die auf Druck reagieren (z.B. Schaltleisten, Schaltmatten) erlauben eine ja/nein Entscheidung und können den Roboter in einen sicheren Zustand überführen. Motorströme und Momentsensoren, z.B. in den Gelenken, der Struktur, den Antrieben, etc. des Roboters, ermöglichen die Messung von Interaktionskräften zwischen Roboter und Umgebung. Eine entsprechende Reaktion des Roboters kann dann eingeleitet werden.

Nahfeldsensoren, wie z.B. kapazitive Sensoren, können eine Änderung des elektrischen Feldes der Sensorumgebung in einem Abstand bis zu einigen Dezimetern erfassen. Aufgrund dieser Änderung kann z.B. ein Rückschluss über die Position des Lebewesens und/oder der weiteren Vorrichtung gemacht werden.

Weitfeldsensoren, wie z.B. eine Kamera, die insbesondere geeignet ist, ein dreidimensionales Bild aufzunehmen, Lichtschranken oder Laserscanner erlauben eine weiträumige Absicherung des Arbeitsbereiches des Roboters.

Ausführungsbeispiele der Erfindung sind exemplarisch in den beigefügten schematischen Zeichnungen dargestellt. Es zeigen:
- Fig. 1: einen medizinischen Arbeitsplatz mit einer Röntgenvorrichtung und
- Fig. 2: einen weiteren medizinischen Arbeitsplatz mit einer Röntgenvorrichtung.

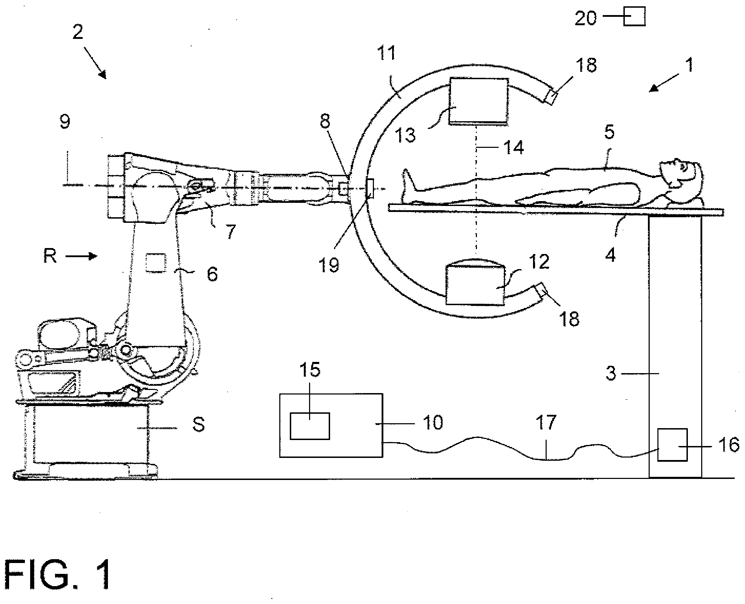

Die Fig. 1 zeigt einen medizinischen Arbeitsplatz 1 mit einer Röntgenvorrichtung 2 und einer im Falle des vorliegenden Ausführungsbeispiels mittels einer Hubvorrichtung 3 höhenverstellbaren Patientenliege 4. Die Patientenliege 4 ist vorgesehen, dass auf ihr ein Lebewesen 5 für eine Untersuchung mit der Röntgenvorrichtung 2 liegen kann.

Die Röntgenvorrichtung 2 weist einen Roboter R mit einer Kinematik für Bewegungen in beispielsweise sechs Freiheitsgraden auf. Der Roboter R weist in allgemein bekannter Weise sechs Bewegungsachsen, Gelenke, Hebel 6, 7 und einen Flansch 8 auf. In der Fig. 1 ist nur eine der Bewegungsachsen mit dem Bezugszeichen 9 versehen. Im Falle des vorliegenden Ausführungsbeispiels ist der Roboter R auf einem Sockel S befestigt.

Jede der Bewegungsachsen 9 wird von einem nicht näher dargestellten Antrieb bewegt. Die Antriebe umfassen beispielsweise jeweils einen elektrischen Motor und Getriebe, wie es dem Fachmann allgemein bekannt ist. Der Roboter R weist ferner einen Steuerrechner 10 auf, der mit den Antrieben des Roboters R in nicht dargestellter Weise verbunden ist und diese in allgemein bekannter Weise mittels eines auf dem Steuerrechner 10 laufenden Rechenprogramms steuert, so dass der Flansch 8 des Roboters R eine vorgegebene Bewegung durchführt.

Die Röntgenvorrichtung 2 weist ferner eine am Flansch 8 des Roboters R befestigte Tragevorrichtung auf, die im Falle des vorliegenden Ausführungsbeispiels als ein C-Bogen 11 ausgeführt ist. Am C-Bogen 11 sind einander gegenüberliegend ein Röntgenstrahler 12 und ein Röntgenstrahlenempfänger 13 angeordnet. Im Falle des vorliegenden Ausführungsbeispiels handelt es sich bei dem Röntgenstrahlenempfänger 13 um einen an sich bekannten Festkörperdetektor. Der Röntgenstrahlenempfänger 13 kann jedoch auch ein Röntgenbildverstärker sein. Im Betrieb der Röntgenvorrichtung 2 trifft eine von dem Röntgenstrahler 12 ausgehende und beim Durchtritt durch das Lebewesen 5 geschwächte Röntgenstrahlung, deren Zentralstrahl 14 in der Fig. 1 dargestellt ist, auf den Röntgenstrahlenempfänger 13 auf. Der Röntgenstrahlenempfänger 13 wandelt die auftreffende Röntgenstrahlung in allgemein bekannter Weise in ein elektrisches Signal um, das einem in den Figuren nicht näher dargestellten und mittels einer in den Figuren ebenfalls nicht näher dargestellten Anzeigeeinrichtung darstellbaren Röntgenbild vom Lebewesen 5 zugeordnet ist.

Im Betrieb der Röntgenvorrichtung 2 kann der C-Bogen 11 mittels des Roboters R insbesondere auf einer vorab festgelegten Bahn bewegt werden.

Im Falle des vorliegenden Ausführungsbeispiels ist auf dem Steuerrechner 10 des Roboters R ein dreidimensionales (3D-) Modell 15 vom medizinischen Arbeitsplatz 1 gespeichert. Das 3D-Modell 15 modelliert die räumliche Ausdehnung der Röntgenvorrichtung 2, also die räumliche Ausdehnung des Roboters R mit dem daran befestigten C-Bogen 11 insbesondere auch währen einer Bewegung des Roboters R. Eine Aktualisierung des 3D-Modells 15 während der Bewegung des Roboters R ergibt sich z.B. aus Signalen von in den Figuren nicht näher dargestellten, dem Fachmann jedoch bekannten Winkelgebern, die die aktuellen Winkel der Bewegungsachsen 9 messen.

Im Falle des vorliegenden Ausführungsbeispiels modelliert das 3D-Modell 15 auch die Patientenliegen 4 und deren Hubvorrichtung 3. Die Hubvorrichtung 3 umfasst z.B. einen elektrischen Antrieb 16, mit dem die Patientenliege 4 in ihrer Höhe verstellbar ist. Der elektrische Antrieb 16 ist im Falle des vorliegenden Ausführungsbeispiels mit einer elektrischen Leitung 17 mit dem Steuerrechner 10 verbunden, so dass der Steuerrechner 10 nicht nur den die Röntgenvorrichtung 2 modellierenden Teil des 3D-Modells 15 aufgrund einer Bewegung des Roboters R aktualisieren kann, sondern auch den die Patientenliege 4 mit Hubvorrichtung 3 modellierenden Teil des 3D-Modells 15 aufgrund einer Höhenverstellung der Patientenliege 4 aktualisieren kann.

Im Falle des vorliegenden Ausführungsbeispiels modelliert das 3D-Modell 15 auch noch das auf der Patientenliege 4 liegende Lebewesen 5, wobei im Steuerrechner 15 die Größe des Lebewesens eingegeben werden kann, um das 3D-Modell 15 dem aktuell auf der Patientenliege 4 liegenden Lebewesen 5 anzupassen. Außerdem kann man angeben, in welcher Position sich das Lebewesen 5 bezüglich der Patientenliege 4 befinden soll, insbesondere ob das Lebewesen 5 auf der Patientenliege 4 liegt.

Im Falle des vorliegenden Ausführungsbeispiels läuft auf dem Steuerrechner 10 ein Rechenprogramm, das aufgrund des 3D-Modells 15 vom medizinischen Arbeitsplatzes 1 überprüft, ob aufgrund der aktuellen Bewegung des Roboters R, der Position der Patientenliege 4 und der Position des C-Bogens 11 eine potenzielle Kollision der Röntgenvorrichtung 2 mit der Patientenliege 4, der Hubvorrichtung 3 oder dem Lebewesen 5 bevorsteht. Erkennt das Rechenprogramm eine solche potenzielle Kollision, dann leitet der Steuerrechner 10 automatische eine geeignete Maßnahme ein, um die potenzielle Kollision zu verhindern oder zumindest deren negative Auswirkung zu verringern. Eine geeignete Maßnahme ist z.B. eine Notbremsung des Roboters R oder eine Änderung der geplanten Bewegung des Roboters R zur Vermeidung der Kollision.

Im Falle des vorliegenden Ausführungsbeispiels sind am C-Bogen 11 zwei Kontaktsensoren 18 angeordnet, die in nicht dargestellter Weise mit dem Steuerrechner 10 verbunden sind. Kommt einer der Kontaktsensoren 18 mit einem Objekt, z.B. mit der Patientenliege 4 oder dem auf der Patientenliege 4 liegenden Lebewesen 5 in Berührung, dann veranlasst der Steuerrechner 10 automatisch, dass der Roboter R seine Bewegung stoppt.

Am C-Bogen 11 ist im Falle des vorliegenden Ausführungsbeispiels auch ein in nicht dargestellter Weise mit dem Steuerrechner 10 verbundener kapazitiver Sensor 19 angeordnet, der eine Änderung eines elektrischen Feldes in der Umgebung des kapazitiven Sensors 19 erkennt. Aufgrund der erkannten Änderung des elektrischen Feldes, der eingestellten Höhe der Patientenliege 4 und des modellierten Lebewesens 5 ist es dem Steuerrechner 10 möglich, u.A. auch eine geänderte Position des Lebewesens 5 zu erkennen, gegebenenfalls eine potenzielle Kollision der Röntgenvorrichtung 2 mit dem Lebewesen 5 zu erkennen und gegebenenfalls die Maßnahme zum Vermeiden der potenziellen Kollision einzuleiten. Aufgrund der erkannten Änderung des elektrischen Feldes ist es dem Steuerrechner 10 auch möglich, eine potenzielle Kollision mit einem weiteren Gegenstand oder einem weiteren Lebewesen zu erkennen, um gegebenenfalls die Maßnahme zum Vermeiden der potenziellen Kollision einzuleiten.

Der medizinische Arbeitsplatz 1 weist im Falle des vorliegenden Ausführungsbeispiels noch einen 3D-Sensor 20 auf, der in nicht dargestellter Weise mit dem Steuerrechner 10 verbunden ist. Der 3D-Sensor 20 ist vorgesehen, ein dreidimensionales Bild vom medizinischen Arbeitsplatz 1 zu erstellen, um insbesondere eine Bewegung des Lebewesens 5 zu erkennen. Aufgrund der erkannten Bewegung des Lebewesens 5 kann der Steuerrechner 10 den das Lebewesen 5 modellierenden Teil des 3D-Modells aktualisieren, wodurch eine potenzielle Kollision der Röntgenvorrichtung 2 mit dem Lebewesen 5 besser erkannt werden kann.

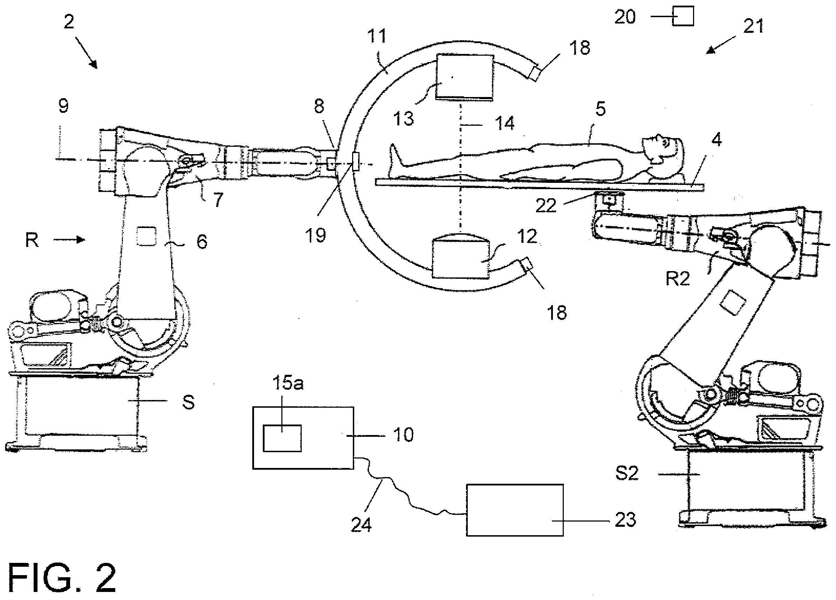

Die Fig. 2 zeigt einen weiteren medizinischen Arbeitsplatz 21. Wenn folgend nicht anders beschrieben, dann sind Bestandteile des medizinischen Arbeitsplatzes 21, die mit Bestandteilen des in der Fig. 1 gezeigten medizinischen Arbeitsplatzes 1 im Wesentlichen funktions- und baugleich sind, mit denselben Bezugszeichen versehen.

Der in der Fig. 2 gezeigte medizinische Arbeitsplatz 21 unterscheidet sich im Wesentlichen von dem in der Fig. 1 gezeigten medizinischen Arbeitsplatz 1 dadurch, dass die Patientenliege nicht mittels der Hubvorrichtung 3, sondern mittels eines Roboters R2 u.A. auch höhenverstellbar ist. Der Roboter R2 ist ähnlich dem Roboter R aufgebaut und weist eine Kinematik für Bewegungen in beispielsweise sechs Freiheitsgraden auf. Der Roboter R2 weist in allgemein bekannter Weise sechs Bewegungsachsen, Gelenke, Hebel und einen Flansch 22 auf und ist im Falle des vorliegenden Ausführungsbeispiels auf einem Sockel S2 befestigt. Am Flansch 22 des Roboters R2 ist die Patientenliege 4 befestigt, so dass der Roboter R2 deren Position verändern kann.

Jede der Bewegungsachsen des Roboters R2 wird von einem nicht näher dargestellten Antrieb bewegt. Die Antriebe umfassen beispielsweise jeweils einen elektrischen Motor und Getriebe, wie es dem Fachmann allgemein bekannt ist. Der Roboter R2 weist ferner einen Steuerrechner 23 auf, der mit den Antrieben des Roboters R2 in nicht dargestellter Weise verbunden ist und diese in allgemein bekannter Weise mittels eines auf dem Steuerrechner 23 laufenden Rechenprogramms steuert, so dass der Flansch 22 des Roboters R2 eine vorgegebene Bewegung durchführt.

Im Steuerrechner 10 des Roboters R ist ein 3D-Modell 15a vom medizinischen Arbeitsplatz 21 gespeichert. Dieses modelliert im Falle des vorliegenden Ausführungsbeispiels die räumliche Ausdehnung der Röntgenvorrichtung 2, also die räumliche Ausdehnung des Roboters R mit dem daran befestigten C-Bogen 11 insbesondere auch während einer Bewegung des Roboters R. Das 3D-Modell 15a modelliert in entsprechender Weise auch die Patientenliegen 4 und den Roboter R2, d.h. deren räumliche Ausdehnungen auch während einer Bewegung des Roboters R2. Damit der Steuerrechner 10 des Roboters R den den Roboter R2 mit Patientenliege 4 modellierenden Teil des 3D-Modells 15a aktualisieren kann, sind die beiden Steuerrechner 10, 23 mittels einer Datenleitung 24 verbunden. Somit kann der Steuerrechner 10 des Roboters R vom Steuerrechner 23 des Roboters R2 eine Information über die aktuellen Winkelstellungen des Roboters R2 erhalten und aufgrund dieser Information das 3D-Modell 15a aktualisieren.

Im Falle des vorliegenden Ausführungsbeispiels modelliert das 3D-Modell 15a auch noch das auf der Patientenliege 4 liegende Lebewesen 5, wobei im Steuerrechner 10 die Größe des Lebewesens 5 eingegeben werden kann, um das 3D-Modell 15a dem aktuell auf der Patientenliege 4 liegenden Lebewesen 5 anzupassen. Außerdem kann man angeben, in welcher Position sich das Lebewesen 5 bezüglich der Patientenliege 4 befindet, insbesondere ob das Lebewesen 5 auf der Patientenliege 4 liegt.

Auch im Falle des vorliegenden Ausführungsbeispiels läuft auf dem Steuerrechner 10 ein Rechenprogramm, das aufgrund des 3D-Modells 15a vom medizinischen Arbeitsplatz 21 überprüft, ob aufgrund der aktuellen Bewegungen der Roboter R, R2, der Position der Patientenliege 4 und der Position des C-Bogens 11 eine potenzielle Kollision der Röntgenvorrichtung 2 mit der Patientenliege 4, dem Roboter R2 oder dem Lebewesen 5 bevorsteht. Erkennt das Rechenprogramm eine solche potenzielle Kollision, dann leitet der Steuerrechner 10 automatische eine geeignete Maßnahme ein, um die potenzielle Kollision zu verhindern oder zumindest deren negative Auswirkung zu verringern. Eine geeignete Maßnahme ist z.B. eine Notbremsung der Roboter R, R2 oder eine Änderung der geplanten Bewegungen der Roboter R, R2 zur Vermeidung der Kollision.

Der medizinische Arbeitsplatz 21 weist im Falle des vorliegenden Ausführungsbeispiels die am C-Bogen 11 angeordneten Kontaktsensoren 18, den am C-Bogen 11 angeordneten kapazitiven Sensor 19 und den 3D-Sensor 20 auf, wodurch eine potenzielle Kollision der Röntgenvorrichtung 2 mit dem Lebewesen 5 besser erkannt werden kann.

Alternativ ist auch möglich, dass ein 3D-Modell 15a des medizinischen Arbeitsplatzes 21 in beiden Steuerrechnern 10, 23 gespeichert ist, wodurch eine redundante Überwachung einer potenziellen Kollision ermöglicht wird.

## Patentansprüche

1. Röntgenvorrichtung für einen medizinischen Arbeitsplatz, aufweisend:
- einen Roboter (R), aufweisend mehrere Achsen (9), eine Steuerungsvorrichtung (10), die eingerichtet ist, die Achsen (9) für eine Bewegung des Roboters (R) anzusteuern, und eine Befestigungsvorrichtung (8), und
- eine an der Befestigungsvorrichtung (8) angeordnete, eine Röntgenstrahlenquelle (12) und einen Röntgenstrahlenempfänger (14) aufweisende Tragevorrichtung (11),
wobei in der Steuerungsvorrichtung (10) ein 3D-Modell (15, 15a) von dem Roboter (R) mit angeordneter Tragevorrichtung (11) gespeichert ist, das die räumliche Ausdehnung des Roboters (R) mit angeordneter Tragevorrichtung (11) während der Bewegung des Roboters (R) modelliert,
wobei das 3D-Modell (15, 15a) auch die räumliche Ausdehnung eines sich innerhalb des medizinischen Arbeitsplatzes (1, 21) befindlichen Lebewesens (5) modelliert und die Steuerungsvorrichtung (10) aufgrund des 3D-Modells (15, 15a) eine potenzielle Kollision der Röntgenvorrichtung (2) mit dem Lebewesen (5) erkennt und den Roboter (R) veranlasst, eine Maßnahme zum Vermeiden der potenziellen Kollision einzuleiten, des Weiteren aufweisend wenigstens einen mit der Steuerungsvorrichtung (10) gekoppelten Sensor (18-20), mittels dem die Umgebung der Röntgenvorrichtung (2) erfasst wird und der eingerichtet ist, eine Bewegung des Lebewesens (5) zu erkennen, um die aktuelle Position des Lebewesens (5) im 3D-Modell (15, 15a) zu aktualisieren.

2. Röntgenvorrichtung nach Anspruch 1, bei der der wenigstens eine Sensor einen Kontaktsensor (18), einen Nahfeldsensor (19) und/oder einen Weitfeldsensor (20) umfasst.

3. Medizinischer Arbeitsplatz, aufweisend
- eine Röntgenvorrichtung (2) nach Anspruch 1, und einen zweiten Roboter (R2) mit angeordneter Patientenliege (4), wobei
- der zweite Roboter (R2) mehrere zweite Achsen, eine zweite Steuerungsvorrichtung (23), die eingerichtet ist, die zweiten Achsen für eine Bewegung des zweiten Roboters (R2) anzusteuern, und eine zweite Befestigungsvorrichtung (22) aufweist,
- die Patientenliege (4) an der zweiten Befestigungsvorrichtung (22) angeordnet ist und
- eine Rechenvorrichtung vorgesehen ist, in der ein 3D-Modell (15a) vom medizinischen Arbeitsplatz (21) gespeichert ist, das sowohl das Modell (15, 15a) des ersten Roboters (R) mit angeordneter Tragvorrichtung (11) und des sich innerhalb des medizinischen Arbeitsplatzes (1, 21) befindlichen Lebewesens (5) enthält,
als auch die räumliche Ausdehnung des zweiten Roboters (R2) mit angeordneter Patientenliege (4) während der Bewegung des zweiten Roboters (R2) modelliert,
wobei die Rechenvorrichtung aufgrund des 3D-Modells (15a) eine potenzielle Kollision der Röntgenvorrichtung (2) mit dem zweiten Roboter (R2) und/oder mit der Patientenliege (4) erkennt und die beiden Roboter (R, R2) veranlasst, eine Maßnahme zum Vermeiden der potenziellen Kollision einzuleiten.

4. Medizinischer Arbeitsplatz nach Anspruch 3, bei dem
- die erste Steuerungsvorrichtung (10) die Rechenvorrichtung umfasst und mit der zweiten Steuerungsvorrichtung (23) gekoppelt ist, oder
- die erste und die zweite Steuerungsvorrichtung (10, 23) jeweils die Rechenvorrichtung umfassen und miteinander gekoppelt sind,
um das 3D-Modell (15a) aufgrund der Bewegungen des ersten und/oder zweiten Roboters (R, R2) zu aktualisieren.

5. Medizinischer Arbeitsplatz nach Anspruch 3 oder 4, bei dem die Rechenvorrichtung aufgrund des 3D-Modells (15a) eine potenzielle Kollision der Röntgenvorrichtung (2), des zweiten Roboters (R2) und/oder der Patientenliege (4) mit dem Lebewesen (5) erkennt und die beiden Roboter (R, R2) veranlasst, eine Maßnahme zum Vermeiden der potenziellen Kollision einzuleiten.

## Claims

1. X-ray device for a medical workstation, comprising:
- a robot (R), having a plurality of axes (9), a control device (10), which is configured to control the axes (9) for a movement of the robot (R), and a fastening device (8), and
- a support device (11) arranged on the fastening device (8) and comprising an X-ray radiation source (12) and an X-ray radiation receiver (14),
wherein in the control device (10) a 3D model (15, 15a) is saved of the robot (R) with arranged support device (11), which models the spatial extension of the robot (R) with arranged support device (11) during the movement of the robot (R),
wherein the 3D model (15, 15a) also models the spatial extension of a living being (5) located within the medical workstation (1, 21), and the control device (10) based on the 3D model (15, 15a) identifies a potential collision of the X-ray device (2) with the living being (5) and causes the robot (R) to initiate a measure for avoiding the potential collision, further having at least one sensor (18-20) coupled to the control device (10), by means of which sensor the area surrounding the X-ray device (2) is detected and which sensor is configured to identify a movement of the living being (5) in order to update the current position of the living being (5) in the 3D model (15, 15a).

2. X-ray device according to claim 1, wherein the at least one sensor comprises a contact sensor (18), a near-field sensor (19) and/or a wide-field sensor (20).

3. Medical workstation, comprising
- an X-ray device (2) according to claim 1, and
- a second robot (R2) with an arranged patient bed (4), wherein
- the second robot (R2) has a plurality of second axes, a second control device (23), which is configured to control the second axes for a movement of the second robot (R2), and a second fastening device (22),
- the patient bed (4) is arranged on the second fastening device (22) and
- a computing device is provided in which a 3D model (15a) of the medical workstation (21) is saved, which contains both the model (15, 15a) of the first robot (R) with arranged support device (11) and the living being (5) located within the medical workstation (1, 21),
and also models the spatial extension of the second robot (R2) with arranged patient bed (4) during the movement of the second robot (R2), wherein the computing device on the basis of the 3D model (15a) identifies a potential collision of the X-ray device (2) with the second robot (R2) and/or with the patient bed (4) and causes the two robots (R, R2) to initiate a measure for avoiding the potential collision.

4. Medical workstation according to claim 3, wherein
- the first control device (10) comprises the computing device and is coupled to the second control device (23), or
- the first and the second control device (10, 23) comprise the computing device respectively and are coupled to one another,
in order to update the 3D model (15a) on the basis of the movements of the first and/or second robot (R, R2).

5. Medical workstation according to claim 3 or 4, wherein the computing device on the basis of the 3D model (15a) identifies a potential collision of the X-ray device (2), of the second robot (R2) and/or the patient bed (4) with the living being (5) and causes the two robots (R, R2) to initiate a measure for avoiding the potential collision.

## Revendications

1. Dispositif de radiographie pour un poste de travail médical, comprenant :
- un robot (R) présentant une pluralité d'axes (9), un dispositif de commande (10) conçu pour piloter les axes (9) pour un mouvement du robot (R), et un dispositif de fixation (8), et
- un dispositif de support (11) monté sur le dispositif de fixation (8) et présentant une source de rayons X (12) et un récepteur de rayons X (14),
dans lequel un modèle 3D (15, 15a) est stocké dans le dispositif de commande (10) par le robot (R) avec le dispositif de support (11) monté, et modélise pendant le mouvement du robot (R) l'étendue spatiale du robot (R) avec le dispositif de support (11) monté,
dans lequel le modèle 3D (15, 15a) modélise également l'extension spatiale d'un être vivant (5) se trouvant à l'intérieur du poste de travail médical (1), et le dispositif de commande (10) détecte, sur la base du modèle 3D (15, 15a), une collision potentielle du dispositif de radiographie (2) avec l'être vivant (5) et fait en sorte que le robot (R) prend une mesure pour éviter la collision potentielle, comprenant en outre au moins un capteur (18-20) couplé au dispositif de commande (10), au moyen duquel l'environnement du dispositif de radiographie (2) est saisi et qui est configuré pour détecter un mouvement de l'être vivant (5) afin d'actualiser la position actuelle de l'être vivant (5) dans le modèle 3D (15, 15a).

2. Dispositif de radiographie selon la revendication 1, dans lequel ledit au moins un capteur comprend un capteur de contact (18), un capteur de champ proche (19) et/ou un capteur de champ lointain (20).

3. Poste de travail médical, comprenant
- un dispositif de radiographie (2) selon la revendication 1, et
- un deuxième robot (R 2) avec un divan d'examen médical (4) monté, dans lequel
- le deuxième robot (R2) comprend une pluralité de deuxièmes axes, un deuxième dispositif de commande (23) conçu pour piloter les deuxièmes axes pour le mouvement du deuxième robot (R2), et un deuxième dispositif de fixation (22),
- le divan d'examen médical (4) est monté sur le deuxième dispositif de fixation (22) et
- un dispositif informatique est prévu, dans lequel est stocké un modèle 3D (15a) du poste de travail médical (21), qui à la fois contient le modèle (15, 15a) du premier robot (R) avec le dispositif de support (11) monté et l'être vivant (5) situé à l'intérieur du poste de travail médical (1, 21),
et aussi
modélise pendant le mouvement du deuxième robot (R2) l'extension spatiale du deuxième robot (R2) avec divan d'examen médical (4) monté, dans lequel
le dispositif informatique détecte, sur la base du modèle 3D (15a), une collision potentielle du dispositif de radiographie (2) avec le deuxième robot (R2) et/ou avec le divan d'examen médical (4) et fait en sorte que les deux robots (R, R2) prennent une mesure pour éviter la collision potentielle.

4. Poste de travail médical selon la revendication 3, dans lequel
- le premier dispositif de commande (10) comprend le dispositif informatique et est couplé au deuxième dispositif de commande (23), ou
- les premier et deuxième dispositifs de commande (10, 23) comprennent chacun le dispositif informatique et sont couplés l'un à l'autre,
afin de mettre à jour le modèle 3D (15a) en fonction des mouvements du premier et/ou du deuxième robot (R, R2).

5. Poste de travail médical selon la revendication 3 ou 4, dans lequel l'appareil informatique détecte, sur la base du modèle 3D (15a), une collision potentielle du dispositif de radiographie (2), du deuxième robot (R2) et/ou du divan d'examen médical (4) avec l'être vivant (5) et fait en sorte que les deux robots (R, R2) prennent une mesure pour éviter la collision potentielle.
